(19) Europäisches Patentamt
European Patent Office
Office européen des brevets

(11) Publication number : **0 502 642 A1**

# (12) EUROPEAN PATENT APPLICATION

(21) Application number : **92301558.0**

(51) Int. Cl.$^5$ : **A61K 9/50**

(22) Date of filing : **25.02.92**

(30) Priority : **07.03.91 GB 9104854**

(43) Date of publication of application :
**09.09.92 Bulletin 92/37**

(84) Designated Contracting States :
**AT BE CH DE DK ES FR GR IT LI LU NL PT SE**

(71) Applicant : **RECKITT AND COLMAN PRODUCTS LIMITED**
**One Burlington Lane**
**London W4 2RW (GB)**

(72) Inventor : **Hansraj, Bashir Ramzanali**
**20 Springdale Way, Beverley**
**North Humberside HU17 8NU (GB)**

(74) Representative : **Allard, Susan Joyce et al**
**BOULT, WADE & TENNANT, 27 Furnival Street**
**London EC4A 1PQ (GB)**

## (54) Sustained release compositions.

(57) Sustained release compositions comprising cores including a pharmacologically active ingredient and having a diameter not greater than 5 mm, which cores are coated with a mixture comprising a) as a film forming agent at least one poly (ethyl acrylate, methyl methacrylate, trimethylammonioethyl methacrylate chloride) and b) at least one anionic surfactant wherein the ratio of a) to b) is from 100 :1 to 10 :1 by weight. Examples of suitable active ingredients are idazoxan, mebeverine, paracetamol and betahistine, and the preferred film forming agents are Eudragit RS and Eudragit RL.

EP 0 502 642 A1

This invention relates to oral sustained release compositions of pharmacologically active compounds.

The problems associated with conventional oral administration of drugs are well known. Thus following oral administration of a drug peak blood level concentrations are attained which then decline until there is repeat administration. To maintain the mean blood level concentrations at a therapeutic level may require either frequent dosing or less frequent dosing at a higher level. The former can result in poor patient compliance whilst with the latter there may be an unacceptable level of side effects.

For the reasons set out above various attempts have been made to produce sustained release dosage forms for orally administered drugs. A number of different approaches have been employed to this end, one of the most commonly used being the application of polymers to coat particles of drug substance. Polymer coats may have two modes of action. If they are soluble under particular conditions of pH they may act to release the drug in particular areas of the gastro-intestinal tract eg only in the small intestine etc, ie delayed or targeted release. If the polymer coats are insoluble or only slowly soluble their permeability may be adjusted to control the ingress of drug solvent (ie gastrointestinal fluids) and the egress of dissolved drug and thus produce sustained release characteristics.

Particularly widely used coating materials of the latter type are the poly(ethyl acrylate, methyl methacrylate, trimethylammonioethyl methacrylate chlorides) of formula I

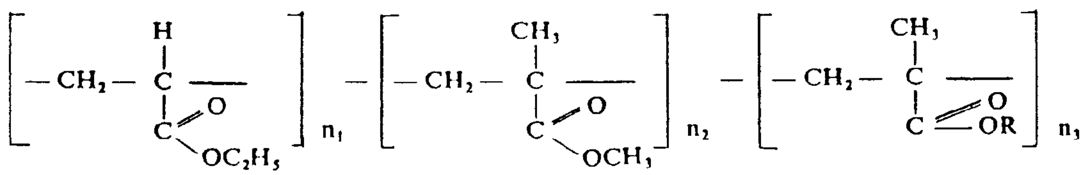

I

where R is $CH_2$-$CH_2$-$N^+(CH_3)_3Cl^-$ and where the ratios of $n_1$:$n_2$:$n_3$ are either 1:2:0.2 or 1:2:0.1 and the total molecular weights are approximately 150,000. These compounds are commercially available as Eudragit RL and Eudragit R5 (Trade Marks of Rohm Pharma). The proportion of $n_3$ type groups controls the degree of permeability of coatings prepared from these compounds as the permeability is directly influenced by the quaternary ammonium groups. Thus various mixtures of RL and RS grade Eudragits may be used to vary the release rates from coated particles.

The use of these Eudragits is not wholly satisfactory because for many soluble drugs, in order to adequately slow down the release rate, rather thick coats are required. This is expensive both in terms of raw materials and spraying time,which can take up to 8 hours or longer. In fact, for some highly soluble drugs, they are not capable of sufficiently slowing the dissolution rate to produce acceptable sustained release dosage forms at all.

We have now found that the release properties of Eudragit RL and RS coats may be substantially modified by the incorporation into the film coats of a relatively small amount of one or more anionic surfactants. This effect was not to be found with non-anionic surfactants tested.

The present invention relates to sustained release compositions comprising cores including a pharmacologically active ingredient and having a diameter not greater than 5mm, which cores are coated with a mixture comprising a) as a film forming agent at least one poly(ethyl acrylate, methyl methacrylate, trimethylammonioethyl methacrylate chloride) and b) at least one anionic surfactant wherein the ratio of a) to b) is from 100:1 to 10:1 by weight.

In an aspect of the invention the ratio of film forming agent to anionic surfactant may be 100:1 to 20:1 by weight.

The present invention also includes a process for producing sustained release compositions of the type hereinbefore described.

Whilst the present invention may be used advantageously with any drug requiring to be given in an orally administered sustained release form, it is particularly useful when used with drugs having high gastro-intestinal solubility, ie aqueous solubility greater than 1g in 100ml, and preferably greater than 1g in 30ml, in the pH range 1 to 7.5.

Suitable active ingredients include idazoxan, cimetidine, dextromethorphan, mebeverine, guaiphenesin, paracetamol, morphine, efaroxan, pseudoephedrine, ranitidine, terfenadine, aspirin, or betahistine; or pharmaceutically acceptable salts thereof.

The sustained release compositions of the present invention may be produced using any type of anionic surfactant, for example alkylaryl sulphonates, alcohol sulphates, sulphosuccinates, sulphosuccinamates, sarcosinates or taurates. Specific examples of these surfactants are sodium lauryl sulphate, dioctyl sodium sulphosuccinate, sodium lauryl sarcosinate and sodium methyl cocoyl taurate.

The cores may consist of pure drug material or may optionally also contain one or more pharmaceutically acceptable excipients such as: fillers/bulking agents eg microcrystalline cellulose; binders eg polyvinyl pyrrolidone; pH stabilisers/buffer systems eg citric acid, sodium bicarbonate etc; sweetening agents eg saccharin; colours; or flavouring agents. The cores may be in the form of pure drug crystals or, more preferably, granules or spheroids.

Granules may be prepared by well known methods essentially consisting of a wet massing phase followed by a drying phase. Drug substance and optionally any excipients are mixed with a solution/suspension of binder to form a wet granular mass. This wet mass is then dried, preferably by fluid bed drying and the granules may optionally be ground to further reduce their size.

Spheroids are produced by the following method. The drug and any excipients are mixed with a sufficient quantity of water to form a 'plastic' wet mass. The mass is extruded into cylinders of uniform diameter and equal length. The extrudates are rolled into spheres using a spheroniser. The spheroids are dried, preferably in a fluid bed drier.

The film coatings comprise the film forming agents (eg Eudragit RL and/or Eudragit RS) plus the anionic surfactants described above in the ratio 100:1 to 10:1. Further conventional excipients may optionally be included in these coats. Well known excipients include: plasticisers such as polyethylene glycols, dibutylphthalate etc; antitack agents such as talc; and pigments.

The film coatings may be prepared by methods well known in the art, particularly by dissolving/dispersing the film forming agents (Eudragits), anionic surfactants and any optional excipients in an appropriate volatile liquid carrier, for example ethanol. The coatings may be applied to the cores by any conventional method, preferably by fluid bed spray coating. The volatile liquid carrier is then removed by evaporation to leave a dry polymer film surrounding the cores.

Conveniently in the sustained release compositions of the present invention the weight of the film forming agent may be 15 to 60% of the weight of the cores and most conveniently 15 to 50% of the weight of the cores.

For convenience of administration and accuracy of dosing the compositions may be in unit dosage form with spheroids or granules filled into, for example, hard gelatine capsules or sachets.

The invention may be further illustrated by reference to the following non-limiting Examples. Comparative Examples are given of the preparation of coated spheroids in which the coating film either contains no surfactant or contains a non-anionic surfactant.

## Example 1

### Coated spheroids containing betahistine

Betahistine-containing spheroid cores were produced to the following formula:

| | % w/w |
|---|---|
| Betahistine dihydrochloride | 20 |
| Avicel PH101 (microcrystalline cellulose) | 80 |

(Avicel is a Trade Mark of FMC, Corporation, USA)

The spheroid cores were produced according to the following method:

Microcrystalline cellulose (1.6Kg) was sieved through a 20 mesh sieve and placed into a Hobart mixer. A 20% w/v solution of betahistine (400g in 2 litres of deionised water) was then added and the mixture was granulated with a further 1.2L of deionised water to form a wet plastic mass. The wet mass was extruded through a perforated screen of 1mm diameter holes using a Nica extruder (Nica System, Sweden). The wet extrudates were spheronised in 700g aliquots using a Nica spheroniser at 650rpm for 5 minutes. The spheroid cores were dried in an Aeromatic Strea 1 fluid bed drier (Niro Atomizer, UK) at a temperature of 50°C for 1 hour, after which they were sieved through a 30 mesh sieve to reject fines and through a 12 mesh sieve to reject agglomerates.

A coating suspension was produced according to the following formula and method:

| | | Units |
|---|---|---|
| Eudragit RS100 | 12 | % w/v |
| Talc | 6 | % w/v |
| Polyethylene glycol 6000 | 1.2 | % w/v |
| Sodium lauryl sulphate | 0.57 | % w/v |
| Deionised water | 2.25 | % w/v |
| Ethanol 96% v/v BP | to 100 | % v/v |

The polyethylene glycol 6000 dissolved in water was added with stirring to the Eudragit dissolved in ethanol and thereafter the sodium lauryl sulphate was added followed by the talc and the resultant mixture made up to volume with ethanol. The ratio of film forming agent to sodium lauryl sulphate was 21:1 by weight.

The dried betahistine spheroid cores were coated with the coating suspension using an Aeromatic Strea 1 fluid bed system set up for top spraying. The spray gun fitted with a 1.2mm aperture nozzle was adjusted to give a narrow angle spray pattern. A peristaltic pump was adjusted to deliver film coating suspension at a rate of about 14ml per minute. Spheroid cores were placed into the coating chamber and prewarmed at an inlet temperature of 30°C. The film coating suspension was placed in a calibrated stainless steel vessel and stirred continuously with an air driven mixer. The spray gun was positioned so as to direct the spray on to the centre of the bed. The atomising pressure was set at 2 bar, and the inlet temperature at 39°C. The peristaltic pump and the timer were started and sufficient coating suspension was sprayed such that, after drying, the weight of film forming agent (ie Eudragit RS100) was 25% of the weight of the spheroid cores. During coating the film coating suspension was continuously stirred with a paddle mixer and the gun checked for any blockages.

When sufficient quantity of coating suspension had been applied, the pump was stopped, the inlet air temperature reduced to 35°C and the atomising air switched off. The spheroids were dried at 30°C for 15 minutes, cooled to 25°C and then sieved through a 30 mesh screen to remove fines and a 12 mesh screen to remove agglomerates. The spheroids were oven dried at 24°C for 7 days to dry off the residual solvent. The weight of film forming agent (ie Eugragit RS100) was checked, and adjusted to 25% of the weight of the spheroid cores if necessary.

Dried coated spheroids were filled into hard gelatine capsules No 0 such that each capsule contained a dose of 24mg betahistine.

Examples 2 to 7 and Comparative Examples A to D

Coated spheroids containing betahistine

Betahistine spheroid cores were produced as in Example 1.

Coating suspensions were produced as in Example 1 with the following variations:

A: No surfactant included.

B: sodium lauryl sulphate replaced with 0.57% w/v tego betain (an amphoteric surfactant).

C: Sodium lauryl sulphate replaced with 0.57% w/v sorbitan polyoxyethylene monooleate (a non-ionic surfactant).

D: Sodium lauryl sulphate replaced with 0.57% w/v cetyl pyridinium chloride (a cationic surfactant).

2: sodium lauryl sulphate replaced with 0.57% w/v dioctyl sodium sulphosuccinate (a sulphosuccinate anionic surfactant).

3: sodium lauryl sulphate replaced with 0.57% w/v sodium lauryl sarcosinate (a sarcosinate anionic surfactant).

4: Sodium lauryl sulphate replaced with 0.57% sodium methyl cocoyl taurate (a taurate anionic surfactant).

5: 0.6% w/v sodium lauryl sulphate used so that the ratio of film forming agent to sodium lauryl sulphate was 20:1 by weight.

6: 0.2% w/v sodium lauryl sulphate used so that the ratio of film forming agent to sodium lauryl sulphate was 60:1 by weight.

7: 0.12% w/v sodium lauryl sulphate used so that the ratio of film forming agent to sodium lauryl sulphate was 100:1 by weight.

The spheroid cores were coated and dried by the methods of Example 1 except for the following variation:
5 to 7: Weight of film forming agent 18.3% of the weight of the spheroid cores.

Dried coated spheroids were filled into hard gelatine capsules No 0 such that each capsule contained a dose of 24mg betahistine.

Example 8

Coated spheroids containing mebeverine

Mebeverine-containing spheroid cores were produced to the following formula:

| | % w/w |
|---|---|
| Mebeverine hydrochloride | 20 |
| Avicel pH 101 | 80 |

The spheroid cores were produced according to the following method:

Avicel (3.2kg) was sieved through a 20 mesh sieve and placed in a 10 litre Fielder mixing bowl (T K Fielder Ltd, UK). The mebeverine hydrochloride (800g) was separately dissolved in 1.5L of deionised water. The impeller blade of the Fielder mixer was set at 200rpm and the mebeverine solution and a further 1.2L of deionised water were added over a 2 minute period. Whilst the impeller blade was left at 200rpm the chopper blade was additionally activated, at the fast reverse setting, for four minutes. The wet mass was extruded and spheronised as in Example 1 but in 1.6kg aliquots. The wet spheroid cores were dried in an Aeromatic Strea 1 fluid bed drier at a temperature of 60°C for 1 hour after which they were sieved through a 30 mesh sieve to reject fines and through a 12 mesh sieve to reject agglomerates.

A coating suspension was produced as in Example 1.

The dried mebeverine spheroid cores were coated and dried using the procedures of Example 1 but sufficient coating suspension was applied such that after drying the weight of the film forming agent was 16% of the weight of the spheroid cores.

Dried coated spheroids were filled into hard gelatine capsules No 0 such that each capsule contained a dose of 50mg mebeverine.

Example 9 and Comparative Examples E and F

Coated spheroids containing mebeverine

Mebeverine spheroid cores were produced as in Example 8.

Coating suspensions were produced as in Example 8 except for the following variation:
E and F: No surfactant included.

The spheroid cores were coated and dried by the methods of Example 8 except for the following variation:
9 and F: Weight of film forming agent 60% of the weight of the spheroid cores.

Dried coated spheroids were filled into hard gelatine capsules No 0 such that each capsule contained a dose of 50mg mebeverine.

Example 10

Coated spheroids containing idazoxan

Idazoxan-containing spheroid cores were produced to the following formula:

| | % w/w |
|---|---|
| Idazoxan HCl | 40 |
| Avicel PH101 | 55 |
| Citric acid | 5 |

The spheroid cores were produced according to the following method:

Idazoxan hydrochloride and microcrystalline cellulose were sieved through a 20 mesh sieve and tumble mixed on a Winkworth mixer (Winkworth Machinery Ltd, England) for 15 minutes. The mixture was granulated with a citric acid solution in a Hobart planetary mixer, model Z200 (Hobart MFG Co Ltd, England), at speed setting 1 for 2 minutes. The wet mass was redistributed and mixed for a further 2 minutes at speed setting 2. The wet mass was extruded through a perforated screen of 1mm diameter holes, using a Nica extruder model E140. The extrudates were spheronised in 700g aliquots at 650rpm for 5 minutes. The wet spheroid cores were dried in an Aeromatic Strea 1 fluid bed drier for 1 hour at 60°C and then sieved through a 30 mesh sieve to reject fines and through a 12 mesh sieve to reject agglomerates.

A coating suspension was produced as in Example 1 but modified by using 9% w/v Eudragit RS100 and 3% w/v RL100 instead of 12% Eudragit RS100. The ratio of film forming agent to sodium lauryl sulphate was 21:1 by weight.

The dried idazoxan spheroid cores were coated and dried using the procedure of Example 1 but sufficient coating suspension was used such that after drying the weight of film forming agent (ie Eudragit RS100 plus Eudragit RL100) was 41.7% of the weight of the spheroid cores.

The dried coated spheroids were filled into hard gelatine capsules No 0 such that each capsule contained a dose of 80mg of idazoxan.

Examples 11 and 12 and Comparative Examples G and H

Coated spheroids containing idazoxan

Idazoxan spheroid cores were produced as in Example 10.

Coating suspensions were produced as in Example 10 except for the following variations:

11: 1.14% w/v sodium lauryl sulphate used such that the ratio of film forming agent to sodium lauryl sulphate was 10.5:1 by weight.

G: No surfactant included.

12: As Example 10 but 12% w/v Eudragit RL100 was used in place of 3% Eudragit RL100 and 9% Eudragit RS100. The ratio of film forming agent to sodium lauryl sulphate was 21:1 by weight.

H: As Example 12 but no surfactant included.

The spheroid cores were coated and dried by the methods of Example 10 except for the following variation:

12 and H: Sufficient coating suspension applied such that after drying the weight of film forming agent was 50% of the weight of the spheroid cores.

Dried coated spheroids were filled into hard gelatine capsules No 0 such that each capsule contained a dose of 80mg of idazoxan.

Example 13

Coated spheroids containing paracetamol

Paracetamol-containing spheroid cores were produced to the following formula:

| | % w/w |
|---|---|
| Paracetamol | 40 |

Example 13 (cont'd)

| | |
|---|---|
| Avicel PH101 | 60 |

The spheroid cores were produced according to the following method:

Avicel pH101 (1.2kg) and paracetamol (800g) were sieved through a 20 mesh sieve and mixed on a Winkworth mixer (Winkworth Machinery Ltd, England) for 20 minutes. Deionised water (2 litres) was then added and the mixture granulated to form a wet plastic mass. The mass was extruded, spheronised, dried and sieved as for Example 1.

A coating suspension was produced as in Example 1.

The dried paracetamol spheroid cores were coated and dried using the procedure of Example 1 but sufficient coating suspension was applied such that after drying the weight of film forming agent was 16% of the weight of the spheroid cores.

Dried, coated spheroids were filled into hard gelatine capsules No 0 such that each capsule contained a dose of 25mg of paracetamol.

Comparative Example J

Coated spheroids containing paracetamol

Paracetamol spheroid cores were produced as in Example 13.

A coating suspension was produced as in Example 13 but no surfactant was included.

The spheroid cores were coated, dried and filled into gelatine capsules as in Example 13.

The release profiles of the coated spheroids of the Examples and Comparative Examples were measured according to the Dissolution Test Method of The United States pharmacopeia XXII (1990) page 1578-1579 using Apparatus 2.Single capsules were added to 900ml of appropriate dissolution fluid (see below) at 37°C and stirred with a paddle rotation speed of 100 rpm. At 20 minutes intervals samples were assayed spectrophotometrically (at an appropriate wavelength, see below) to determine the amount of drug in the dissolution fluid. The percentage drug released from the capsule was calculated and the mean of at least six capsules used to calculate the mean percentage drug released.

Betahistine-containing spheroids (ie Examples 1 to 7 and Comparative Examples A to D) were tested using pH 6.8 phosphate buffer as the dissolution fluid and the drug release was measured at a wavelength of 262nm.

Mebeverine-containing spheroids (ie Examples 8 and 9 and Comparative Examples E and F) were tested using 0.1 Molar hydrochloric acid as the dissolution fluid and the drug release was measured at a wavelength of 266nm.

Idazoxan-containing spheroids (ie Examples 10, 11 and 12 and Comparative Examples G and H) were tested using 0.1 Molar hydrochloric acid as the dissolution fluid and the drug release was measured at a wavelength of 274nm.

Paracetamol- containing spheroids (ie Example 13 and Comparative Example J) were tested using 0.1 Molar hydrochloric acid as the dissolution fluid and the drug release was measured at a wavelength of 248nm.

Mean percentage drug released over time is shown graphically for all the Examples in Figures 1 to 7.

Figure 1 shows the mean percentage drug released during testing for Example 1 and comparative Examples A, 8, C and D. Example 1 (containing sodium lauryl sulphate in the film coating) has a much slower release rate than Comparative Example A (no surfactant in the film coat) or Comparative Examples B, C and D (containing amphoteric, nonionic and cationic surfactants respectively) and in fact approximates to zero order release. The three non-anionic surfactant Comparative Examples (8, C and D) do not exhibit release rates significantly different to that of Comparative Example A which contains no surfactant.

Figure 2 shows the mean percentage drug released during testing for Examples 1, 2, 3 and 4 and Comparative Example A. All four Examples, which contain an alcohol sulphate, a sulphosuccinamate, a sarcosinate and a taurate surfactant (ie all anionic surfactants) respectively, have slower release rates than the Comparative Example A, which contains no surfactant.

Figure 3 shows the mean percentage drug released during testing for Examples 5, 6 and 7 (which have Eudragit to surfactant ratios of 20:1, 60:1 and 100:1 respectively). These Examples illustrate how the release rate may be varied for a particular drug by altering the amount of anionic surfactant but maintaining a constant concentration of coating material.

Figure 4 shows the mean percentage drug released during testing for Examples 8 and 9 (having Eudragit contents of 16% and 60% by weight of the spheroid cores respectively) and Comparative Examples E and F

(having equivalent coating levels to Examples 8 and 9, but containing no surfactants). Both Examples have slower release rates than their respective Comparative Examples and this difference is most marked at lower coating levels (Example 8 versus Comparative Example E).

## Claims

1. Sustained release compositions comprising cores including a pharmacologically active ingredient and having a diameter not greater than 5 mm, which cores are coated with a mixture comprising a) as a film forming agent at least one poly(ethyl acrylate, methyl methacrylate, trimethylammonioethyl methacrylate chloride) and b) at least one anionic surfactant wherein the ratio of a) to b) is from 100:1 to 10:1 by weight.

2. Sustained release compositions as claimed in claim 1 in which the ratio of a) to b) is from 100:1 to 20:1 by weight.

3. Sustained release compsotions as claimed in claim 1 or claim 2 in which the pharmacologically active ingredient has an aqueous solubility of greater than 1g in 100 ml in the pH range 1 to 7.5.

4. Sustained release compositions as claimed in any one of the preceding claims in which the pharmaceutically active ingredient is idazoxan, cimetidine, dextromethorphan, mebeverine, guaiphenesin, paracetamol, morphine, efaroxan, pseudoephedrine, ranitidine, terfenadine, aspirin or betahistine; or a pharmaceutically acceptable salt thereof.

5. Sustained release compositions as claimed in claim 4 in which the pharmaceutically active ingredient is idazoxan, mebeverine, paracetamol or betahistine; or a pharmaceutically acceptable salt thereof.

6. Sustained release compositions as claimed in any one of the preceding claims in which the cores further include at least one pharmaceutically acceptably excipient.

7. Sustained release compositions as claimed in any one of the preceding claims in which the anionic surfactant is an alkylaryl sulphonate, an alcohol sulphate, a sulphosuccinate, a sulphosuccinamate, a sarcosinate or a taurate.

8. Sustained release compositions as claimed in claim 7 in which the anionic surfactant is sodium lauryl sulphate, dioctyl sodium sulphosuccinate, sodium lauryl sarcosinate or sodium methyl cocoyl taurate.

9. Sustained release compositions as claimed in any one of the preceding claims in which the weight of the film forming agent is 15 to 60%, preferably 15 to 50%, of the weight of the cores.

10. Sustained release compositions as claimed in any one of the preceding claims in which the cores are in the form of spheroids or granules.

11. Sustained release compositions in unit dosage form comprising the spheroids or granules of claim 10 filled into hard gelatine capsules or sachets.

## Claims for the following Contracting States: ES, GR

1. A process for producing a sustained release composition of a pharmacologically active ingredient comprising forming said active ingredient and optionally at least one pharmaceutically acceptable excipient into cores of not more than 5mm diameter and coating said cores with a mixture comprising a) as a film forming agent at least one poly (ethyl acrylate, methyl methacrylate, trimethylammonioethyl methacrylate chloride) and b) at least one anionic surfactant wherein the ratio of a) to b) is from 100:1 to 10:1 by weight.

2. A process as claimed in claim 1 in which the ratio of a) to b) is from 100:1 to 20:1 by weight.

3. A process as claimed in claim 1 or claim 2 wherein the coating is applied by spray coating.

4. A process as claimed in any one of claims 1 to 3 in which the pharmacologically active ingredient has an aqueous solubility of greater than 1g in 100 ml in the pH range 1 to 7.5.

**5.** A process is claimed in any one of claims 1 to 4 in wich the pharmacologically active ingredient is idazoxan, cimetidine, dextromethorphan, mebeverine, guaiphenesin, paracetamol, morphine, efaroxan, pseudoephedrine, ranitidine, terfenadine, aspirin or betahistine; or a pharmaceutically acceptable salt thereof.

**6.** A process as claimed in claim 5 in which the pharmaceutically active ingredient is idazoxan, mebeverine, paracetamol or betahistine; or a pharmaceutically acceptable salt thereof.

**7.** A process as claimed in any one of claim 1 to 6 in which the anionic surfactant is an alkylaryl sulphonate, an alcohol sulphate, a sulphosuccinate, a sulphosuccinamate, a sarcosinate or a taurate.

**8.** A process as claimed in claim 7 in which the anionic surfactant is sodium lauryl sulphate, dioctyl sodium sulphosuccinate, sodium lauryl sarcosinate or sodium methyl cocoyl taurate.

**9.** A process as claimed in any one of claims 1 to 8 in which the weight of the film forming agent is 15 to 60%, preferably 15 to 50%, of the weight of the cores.

**10.** A process as claimed in any one of claims 1 to 9 in which the cores are in the form of granules or spheroids.

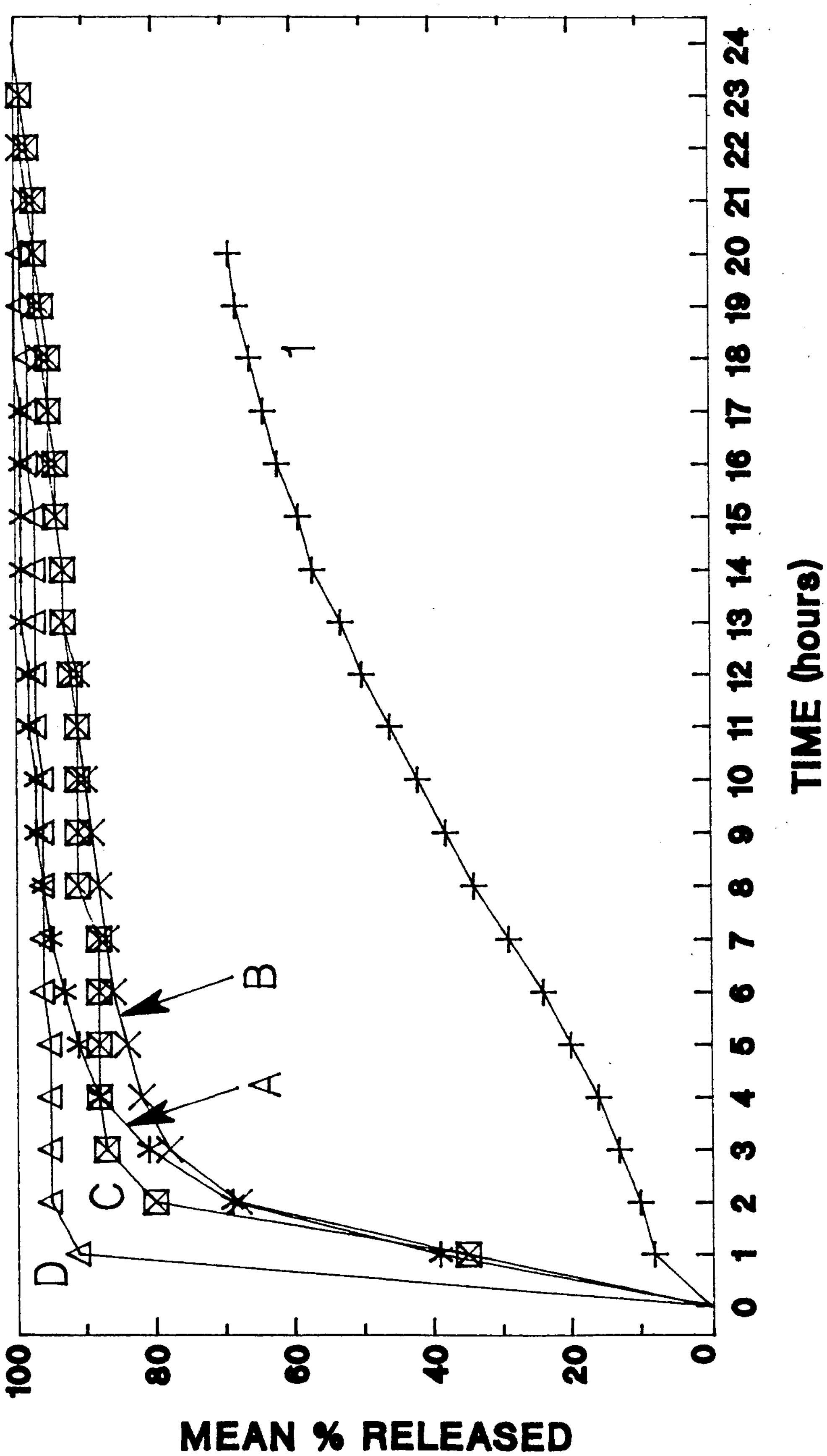
MEAN % RELEASED
0
20
40
60
80
100
TIME (hours)
0 1 2 3 4 5 6 7 8 9 10 11 12 13 14 15 16 17 18 19 20 21 22 23 24
A
B
C
D
1

FIGURE 1

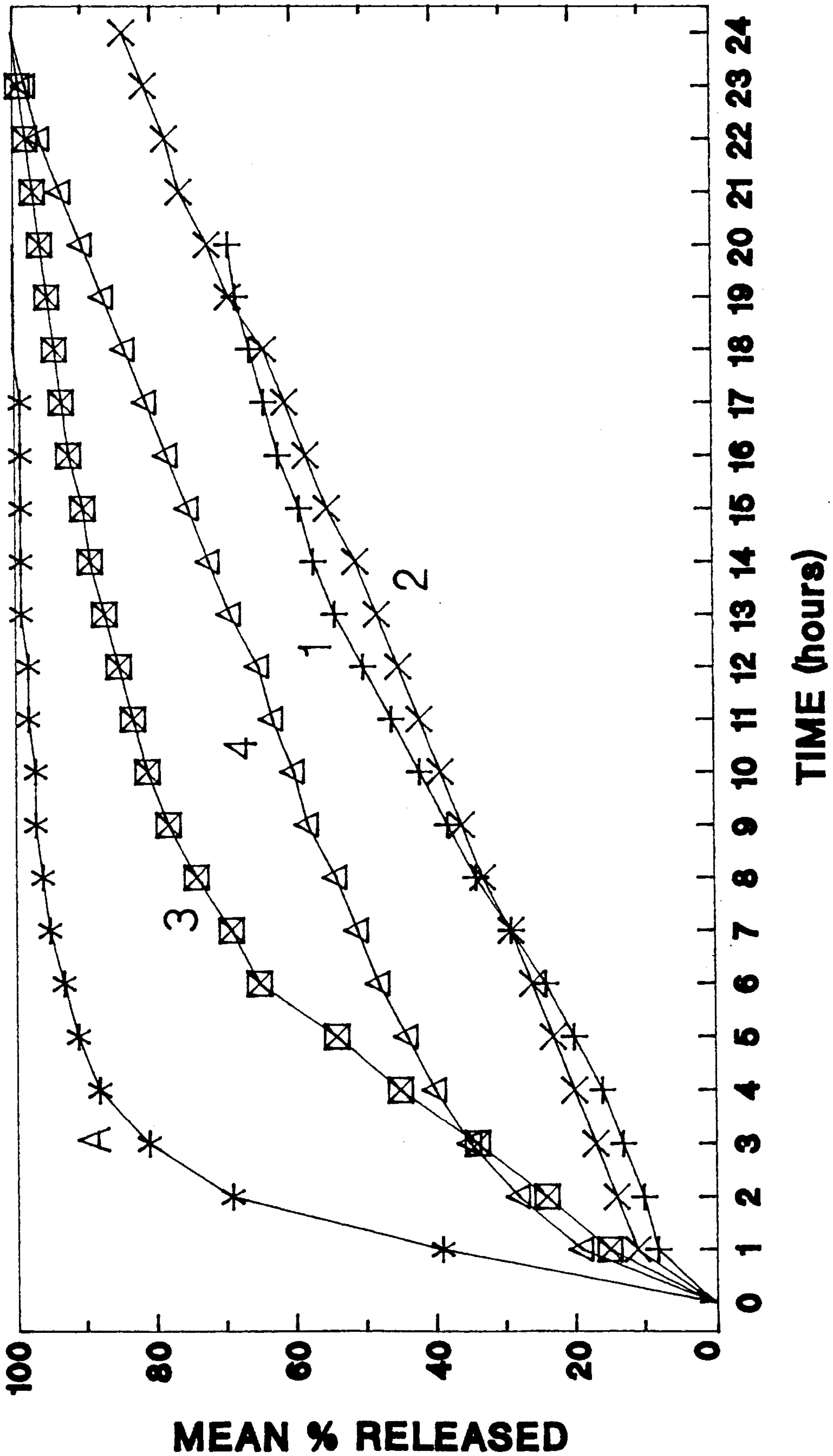
MEAN % RELEASED
0
20
40
60
80
100
TIME (hours)
0
1
2
3
4
5
6
7
8
9
10
11
12
13
14
15
16
17
18
19
20
21
22
23
24
A
1
2
3
4

FIGURE 2

FIGURE 3

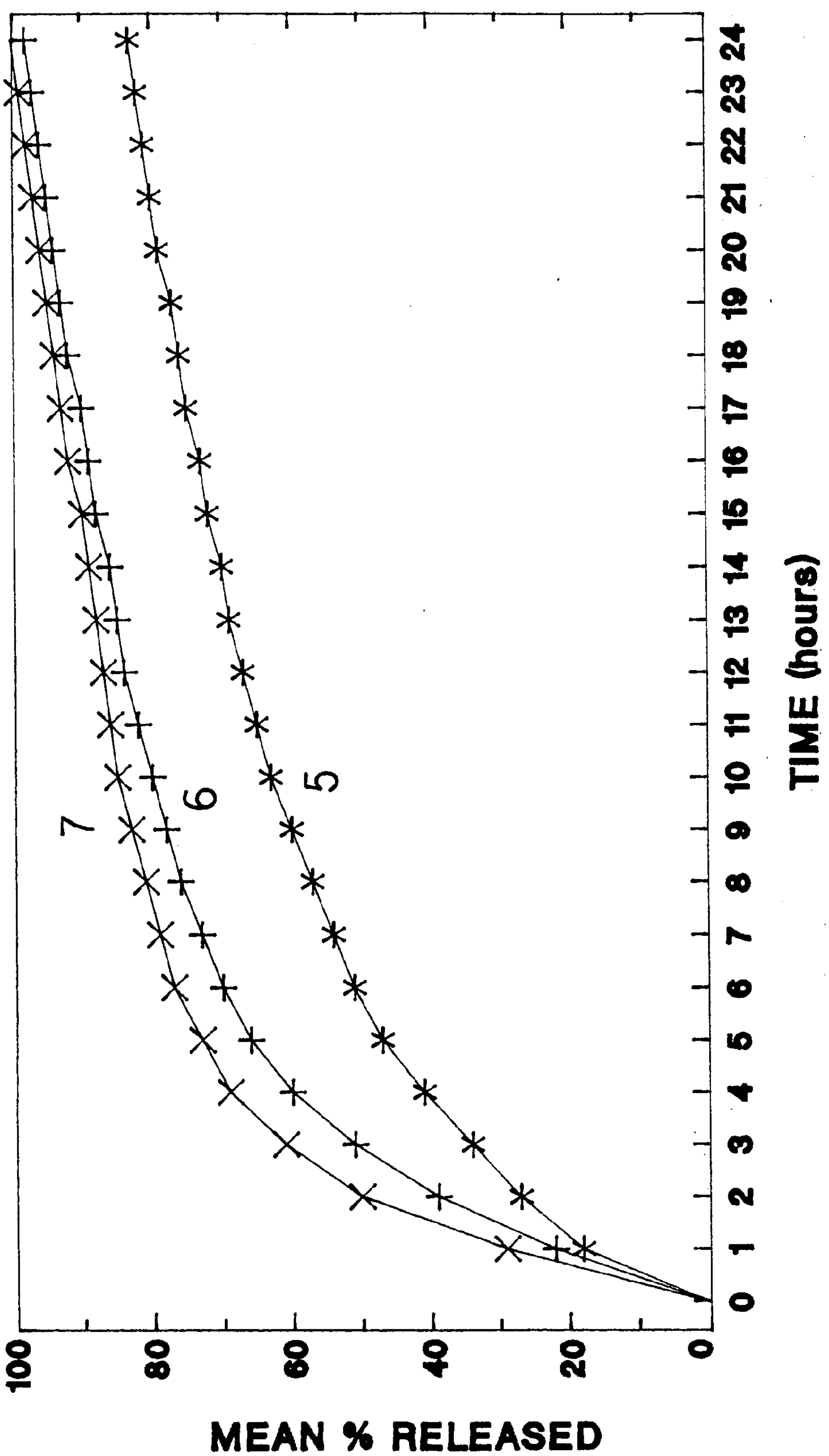
MEAN % RELEASED
0
20
40
60
80
100
TIME (hours)
0 1 2 3 4 5 6 7 8 9 10 11 12 13 14 15 16 17 18 19 20 21 22 23 24
5
6
7

FIGURE 4

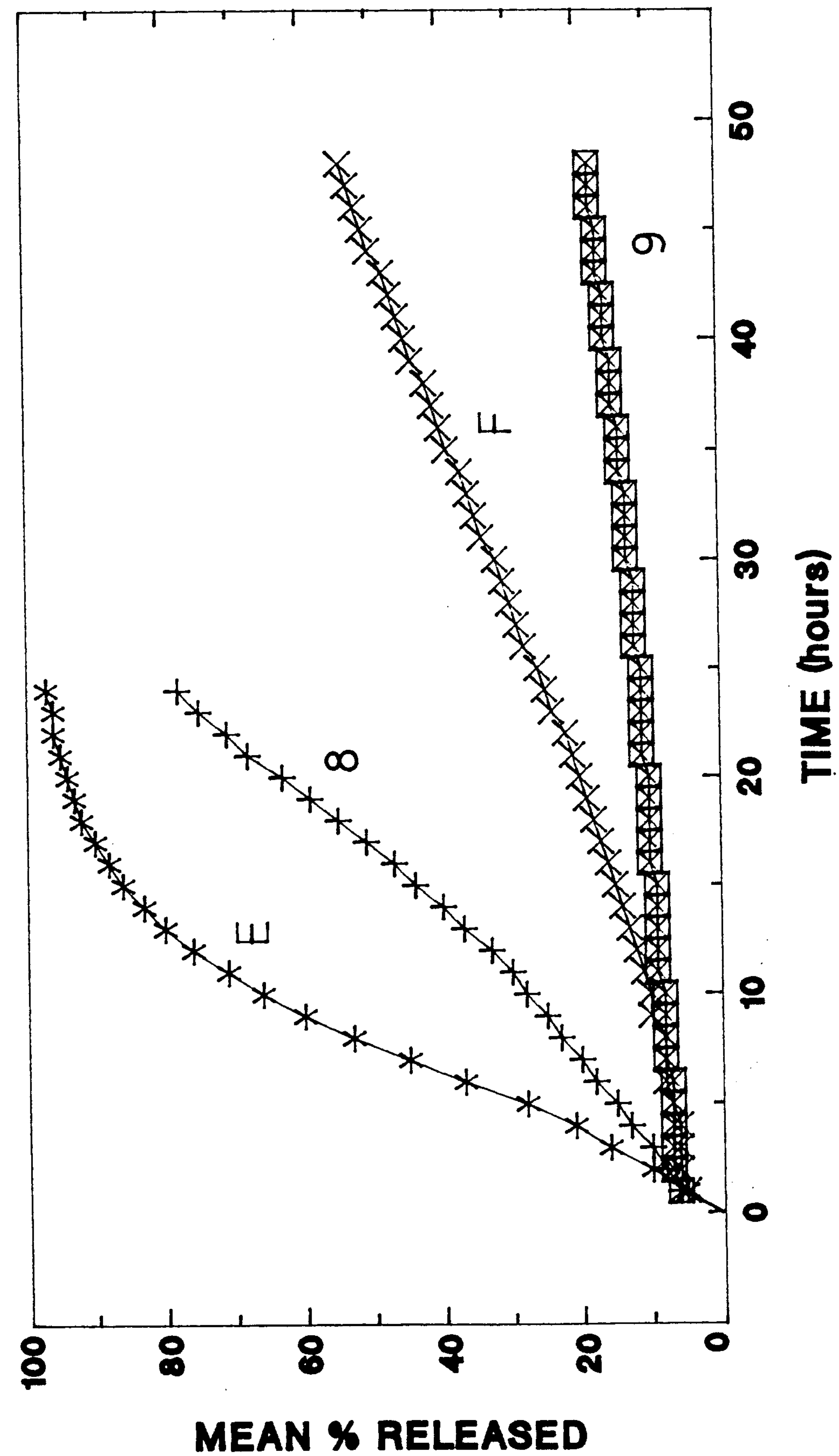

FIGURE 5

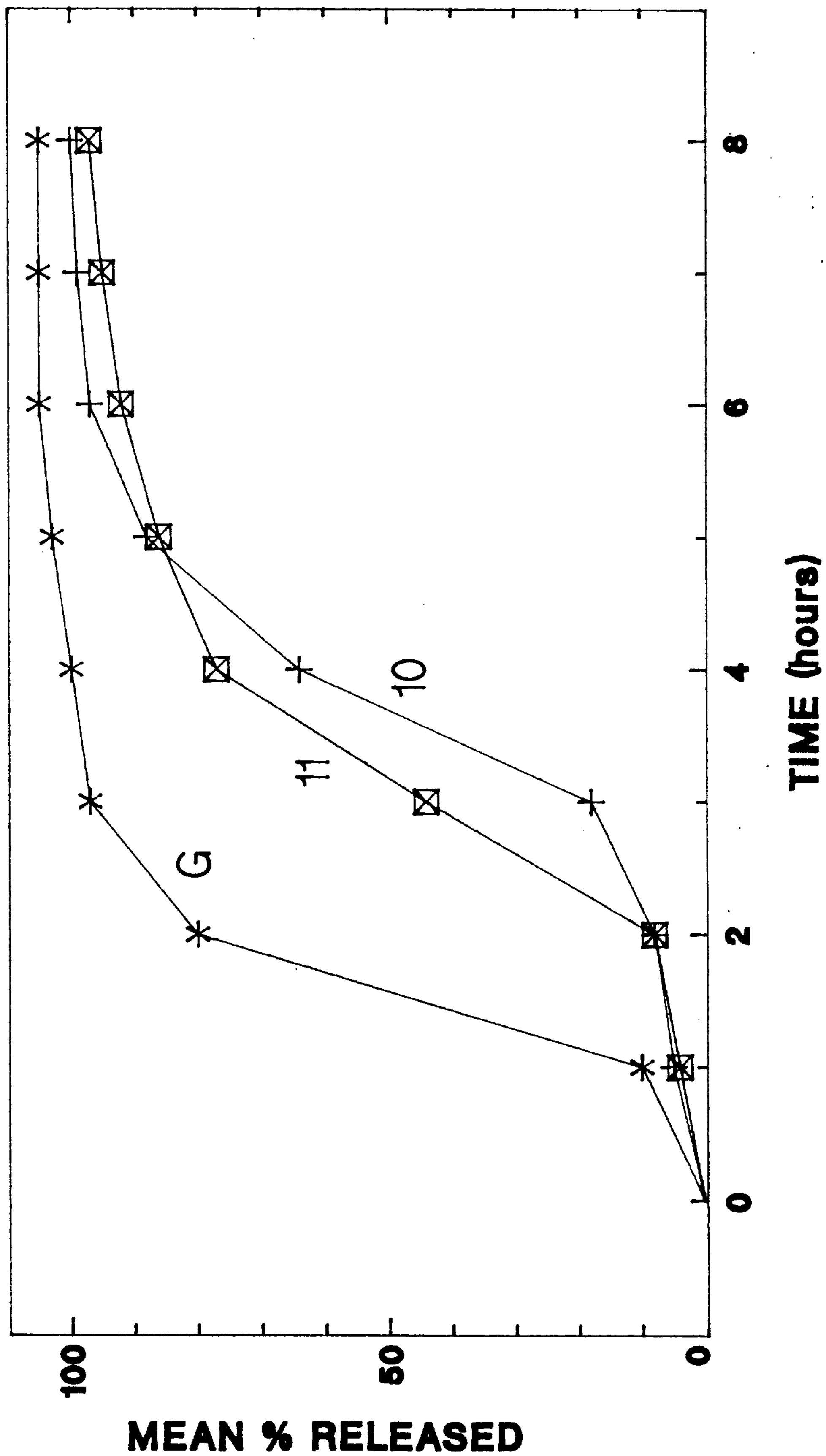

FIGURE 6

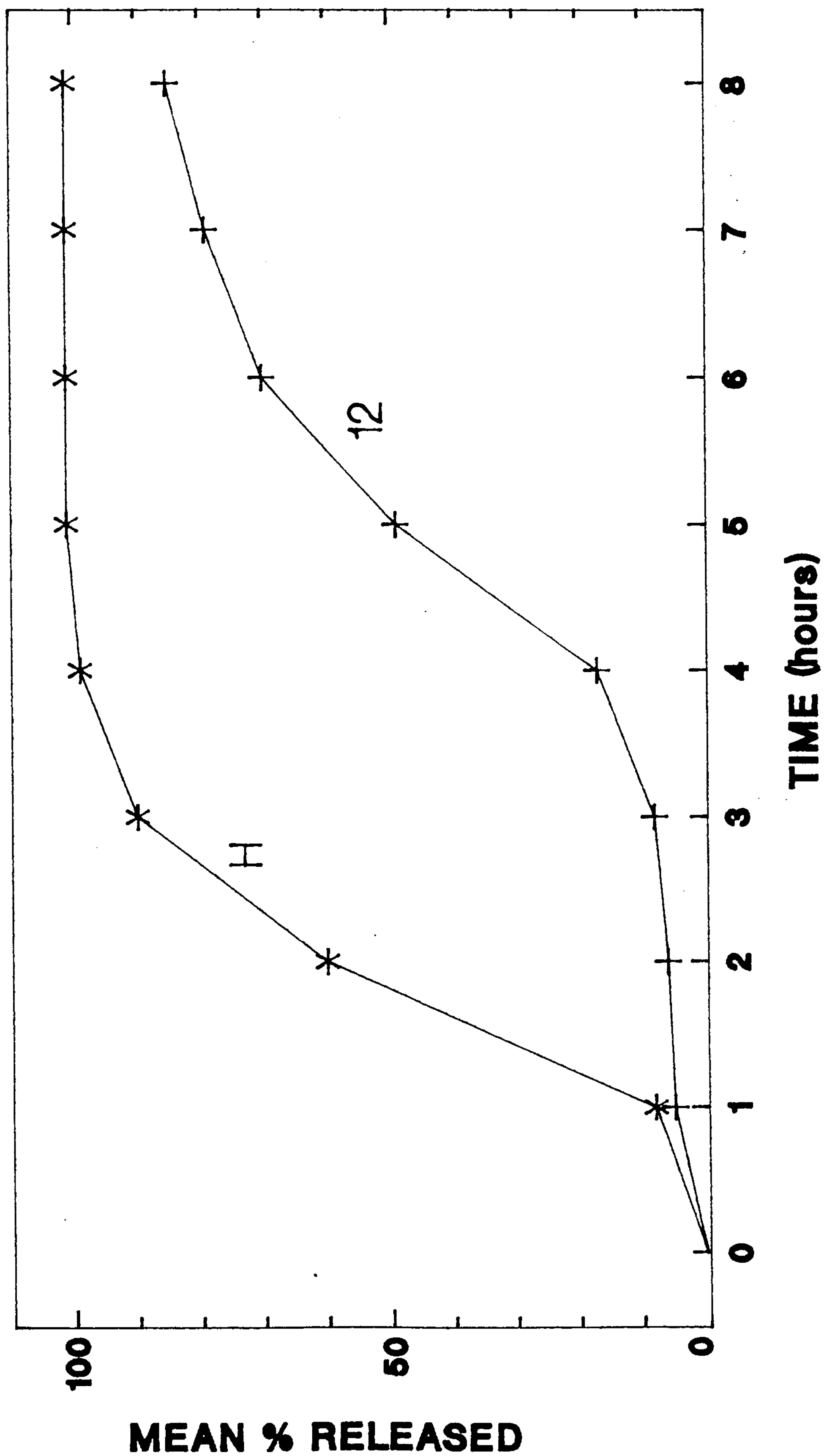

FIGURE 7

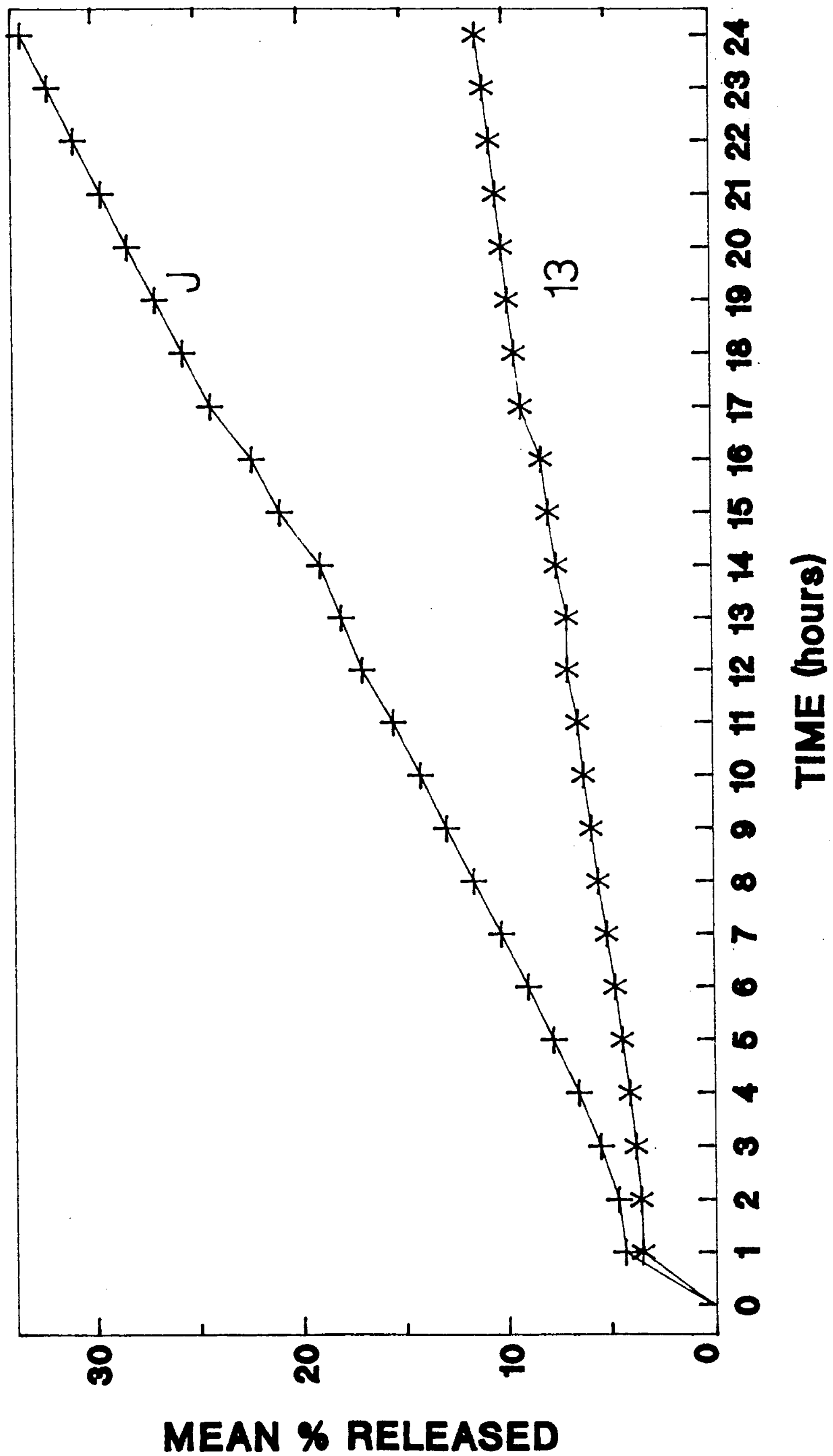
MEAN % RELEASED
0
10
20
30
TIME (hours)
0 1 2 3 4 5 6 7 8 9 10 11 12 13 14 15 16 17 18 19 20 21 22 23 24
J
13

European Patent Office

# EUROPEAN SEARCH REPORT

Application Number

EP 92 30 1558

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 080 341 (A/S ALFRED BENZON) * Claims 1,2,5,12-17,20-26,30; page 2, line 30 - page 3, line 4; page 4, line 28 - page 5, line 3; page 5, lines 22-29; page 6, lines 7-11; page 8, line 29 - page 9, line 5; page 12, lines 1-11; page 13, lines 8-11 * | 1-8,10-11 | A 61 K 9/50 |
| Y | US-A-4 871 546 (D.R. FELTZ et al.) * Claim 2; column 1, lines 39-55; column 2, lines 19-21,40-41 * | 1-8,10-11 | |
| A | FR-A-2 404 029 (SANKYO CO., LTD) * Claims 1-3,7,12,13; page 2, lines 27-35; page 3, lines 22-36; page 4, lines 34-37; page 5, lines 5-13; page 7, lines 8-14 * | 1-8,10-11 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 10-06-1992 | VENTURA AMAT A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)